# EUROPEAN PATENT APPLICATION

(11) **EP 4 789 622 A1**
(43) Date of publication of application: **12.08.2026**
(21) Application number: 25214338.3
(22) Date of filing: 30.01.2025
(51) Int. Cl.: A61B 8/06, A61B 5/00, A61B 8/00, A63B 22/00, A63B 24/00, G16H 50/30

(54) **WEARABLE FITNESS DEVICES AND METHODS**

(62) Document number(s) of the earlier application(s) in accordance with Art. 76 EPC: 25154950.7 / 4 785 873
(71) Applicant: SonoFit AS, 7013 Trondheim (NO)
(72) Inventor: Frigstad, Sigmund, 7014 Trondheim (NO)
(74) Representative: Håmsø Patentbyrå AS

(57) **Abstract**

A device for determining in use when a user is exercising below, at or above the anaerobic threshold, the device comprising an ultrasound transducer and a processor wherein: the ultrasound transducer is signal connected to the processor and the ultrasound transducer is arranged in use to: be attached to the body of the user before and during exercise; send a series of ultrasound waves at a doppler angle of between 0 and 80 degrees to a blood flow in the body before exercise begins; receive a first series of ultrasound wave reflections back from the blood flow with a doppler shift; and send a series of ultrasound waves at the same doppler angle of between 0 and 80 degrees to a blood flow in the body during exercise; receive a second series of ultrasound wave reflections back from the blood flow with a doppler shift; and send the ultrasound wave data to the processor; wherein the processor is configured to: receive ultrasound wave data from the ultrasound transducer and: determine, at a first period in the cardiac phase, the doppler shift of the series of ultrasound waves sent to the blood flow before exercise; determine, at a second period in the cardiac phase corresponding to the first period in the cardiac phase, the doppler shift of the series of ultrasound waves sent to the blood flow during exercise; and calculate a velocity growth factor based on the doppler shifts at the first and second corresponding periods in the cardiac phases; and compare the velocity growth factor with a predetermined anaerobic velocity growth factor or a predetermined anaerobic velocity growth factor range; and determine that the user is: exercising below the anaerobic threshold if the velocity growth factor is less than the anaerobic velocity growth factor or less than the lower limit of the anaerobic velocity growth factor range; or exercising at the anaerobic threshold if the velocity growth factor is equal to the anaerobic velocity growth factor or within the anaerobic velocity growth factor range; or exercising above the anaerobic threshold if the velocity growth factor is greater than the anaerobic velocity growth factor or greater than the upper limit of the anaerobic velocity growth factor range.

## Description

### FIELD

The present invention relates to a fitness devices and associated methods. More specifically, the present invention relates to wearable fitness devices and methods related to monitoring a physical property of the human body and reporting useful information to improve physical training. More specifically, the present invention relates to devices and methods for determining when the user is exercising below the anaerobic threshold, at the anaerobic threshold or above the anaerobic threshold and to devices and methods for determining the anaerobic threshold of a user.

### BACKGROUND

Monitoring of the human body to guide training and performance in sport is very common, particularly among top athletes. To maximise training and/or performance, it is well known to test and monitor physical attributes of the human body before, during and after training and during rest periods such as sleep. In high intensity sports such as running and cycling, the lactate threshold and anaerobic threshold are important.

The lactate threshold and the anaerobic threshold are related concepts in exercise physiology, but they are not identical and are often confused while being, for many purposes, similar enough to be considered the same threshold, as will be explained.

The lactate threshold is the exercise intensity at which lactate begins to accumulate in the blood at a faster rate than it can be cleared. At low exercise intensities, lactate is produced at a low rate and is cleared efficiently. As intensity increases, lactate production rises. The lactate threshold marks the point where this balance tips, indicating a shift toward greater reliance on anaerobic metabolism. The significance of the lactate threshold in training is that it represents the highest intensity an athlete can sustain for a prolonged period without fatigue and lactate build up.

The anaerobic threshold is the exercise intensity at which anaerobic energy pathways start to contribute significantly to overall energy production, often leading to a rapid accumulation of lactate and a shift toward fatigue. The anaerobic threshold occurs at a slightly higher intensity than the previously discussed lactate threshold.

In some research, the lactate threshold and anaerobic threshold are considered nearly synonymous, in other research, the anaerobic threshold is tied more to ventilatory or metabolic changes and may not always align precisely with the lactate threshold.

The so called "Norwegian model" of lactate threshold monitoring is a systematic approach to monitoring and optimising endurance training based on lactate measurements. It is widely associated with Norwegian cross-country skiers and distance runners, such as Jakob Ingebrigtsen and the Ingebrigtsen family, and has been popularized by their coaches, including Gjert Ingebrigtsen.

The Norwegian model emphasizes precise control of training intensity and uses lactate threshold data as a cornerstone for individualized training plans. The key features of the Norwegian model are now described. Most importantly for the subject-matter described in the present application, the Norwegian model features frequent lactate testing during training. Athletes measure blood lactate levels during workouts, often by taking small blood samples from the fingertip or ear using portable lactate analysers. Testing is conducted in real time to ensure the athlete stays within desired lactate zones. This has several drawbacks.

Firstly, invasive blood sampling can cause mild pain or discomfort, especially if repeated frequently during training. For individuals with sensitive skin, the repeated pricking of the same area may result in tenderness, soreness, or irritation. While lower than with larger needles, the break in the skin barrier still poses a risk of infection, particularly if hygiene protocols (e.g., cleaning the skin and lancet use) are not strictly followed. Applying too much or too little pressure during sampling can alter blood composition, leading to variability in lactate concentration. Changes in sampling location (e.g., fingertip vs. earlobe) might produce slight differences in results. Repeated pinpricks during an activity, such as endurance training, may feel disruptive and uncomfortable over time. Collecting blood samples outdoors or in non-controlled environments might be less convenient or hygienic. For individuals sensitive to the sight of blood or needles, even small pinpricks may cause anxiety or stress. Reusing lancets or improper disposal can lead to contamination, though this is rare with proper protocols. Failure to clean the sampling area properly may result in contamination of the sample or infection. While a single pinprick is minimally invasive, repeated sampling-especially over weeks or months-can cause cumulative irritation or scarring in the same areas.

Another disadvantage of the described Norwegian model is that sampling is not continuous during exercise and is instead taken after a training interval. Further, the test strips required are relatively expensive, especially when it is considered that the strips cannot be reused. Although the test strips produce a relatively small amount of waste, it should also be noted that the non-reusable test strips are not environmentally friendly.

Finally, performing accurate and proper testing requires a trained assistant to perform proper calibration and handling of the device and test strips, and to perform proper cleaning and maintenance.

Training regimes utilising the Norwegian model are typically designed to maintain lactate levels in a specific range that maximises aerobic development while minimizing fatigue. The most common focus is on Lactate Threshold 1 (LT1) and Lactate Threshold 2 (LT2). LT1 is the point where lactate starts to accumulate in the blood, marking the transition from easy to moderate exercise. LT2 is the point where lactate production exceeds clearance, marking the transition to high-intensity exercise. According to the Norwegian model, training is divided into lower-intensity aerobic sessions and higher-intensity threshold work, with a balance designed to prevent overtraining and ensure continuous improvement. Athletes often train slightly below or around LT2 during threshold sessions. Lactate measurements help tailor training to the athlete's unique physiology. The Norwegian model avoids a one-size-fits-all approach by relying on precise, data-driven adjustments.

Endurance athletes often train at high volumes, with a significant portion of the training at low intensity. The Norwegian model emphasises avoiding "no man's land" training - moderate intensities that are too hard to recover from but not hard enough to provide significant physiological stimulus.

As previously explained, the anaerobic threshold is a theoretical point where the body transitions from primarily aerobic energy production to a state where anaerobic metabolism contributes significantly. This corresponds to a higher exercise intensity where oxygen delivery or utilisation cannot meet the energy demands, resulting in increased lactate production. The anaerobic threshold does not have a direct measurable counterpart, whereas lactate threshold is a specific and measurable physiological marker, which can be tested as described above.

In practice, the lactate threshold is often used as a proxy for the anaerobic threshold because lactate accumulation reflects the increasing reliance on anaerobic energy systems.

For most athletes and training purposes, lactate threshold and anaerobic threshold are treated similarly because they both signify a critical intensity beyond which exercise becomes much more physiologically demanding.

Aerobic exercise is physical exercise under conditions where the muscles have enough oxygen, and energy is created by oxidation of carbohydrates and fats. This is the primary method of energy production during prolonged, low to moderate-intensity activities.

When the exercising intensity is high and the blood is not able to transport enough oxygen to the muscles, glycolysis becomes the main source of energy production. Glycolysis does not require oxygen hence anaerobic. Instead, glucose is broken down to lactate resulting in rapid production of adenosine triphosphate (ATP). This anaerobic exercise can only be sustained for short periods because it relies on energy sources that are quickly depleted. It will also result in a rapid build-up of lactate which will cause muscle soreness and take time to recover from.

The benefits of the above-described threshold training are to sustain higher intensity for longer periods of time without the soreness and fatigue from anaerobic exercise while enhancing overall endurance.

Along with the already described measurement of lactate using blood sampling, it is also possible to find the anaerobic threshold by measuring the gas concentration of oxygen and carbon dioxide in the exhalation air. When the carbon dioxide to oxygen ratio, also called the respiratory exchange rate (RER), becomes larger than 1.0, anaerobic metabolism starts to dominate. However, oxygen measurements require a mask during the athletic activity, such as running, therefore this technique is less in use by athletes during training, it is mainly used for research purposes.

Development is also going on to measure the lactate concentration from sweat, but results are highly variable, and the method is not in use by athletes yet.

It is also possible to estimate the anaerobic threshold as a certain percentage (85-90%) of the maximum heart rate, but this is very inaccurate and requires measurements of maximum heart rate (HRmax). Many smart watches / fitness watches provide a rough estimation of the anaerobic threshold using a maximum heart rate value calculated as a percentage of the user's age-predicted maximum heart rate.

In summary, threshold training is an effective training method for athletes. However, blood sampling and breath concentration monitoring have significant drawbacks as outlined above. More simple methods, for example based on estimated maximum heart rate, are unreliable, particularly for performance athletes.

There is therefore a need for devices and methods to determine the user's anaerobic threshold. There is also a need for devices and methods that, when the user's anaerobic threshold is known, can inform the user when the user is exercising below the anaerobic threshold, at or around the anaerobic threshold or above the anaerobic threshold. There is also a need for devices and methods to determine the user's anaerobic threshold and inform the user when the user is exercising below the anaerobic threshold, at or around the anaerobic threshold or above the anaerobic threshold.

At least one aim of the invention is to obviate or at least mitigate one or more drawbacks of prior art.

### SUMMARY

According to a first aspect of the invention, there is provided a device for determining in use when a user is exercising below, at or above the anaerobic threshold, the device comprising an ultrasound transducer and a processor wherein: the ultrasound transducer is signal connected to the processor and the ultrasound transducer is arranged in use to: be attached to the body of the user before and during exercise; send a series of ultrasound waves at a doppler angle of between 0 and 80 degrees to a blood flow in the body before exercise begins; receive a first series of ultrasound wave reflections back from the blood flow with a doppler shift; and send a series of ultrasound waves at the same doppler angle of between 0 and 80 degrees to a blood flow in the body during exercise; receive a second series of ultrasound wave reflections back from the blood flow with a doppler shift; and send the ultrasound wave data to the processor; wherein the processor is configured to: receive ultrasound wave data from the ultrasound transducer and: determine, at a first period in the cardiac phase, the doppler shift of the series of ultrasound waves sent to the blood flow before exercise; determine, at a second period in the cardiac phase corresponding to the first period in the cardiac phase, the doppler shift of the series of ultrasound waves sent to the blood flow during exercise; and calculate a velocity growth factor based on the doppler shifts at the first and second corresponding periods in the cardiac phases; and compare the velocity growth factor with a predetermined anaerobic velocity growth factor or a predetermined anaerobic velocity growth factor range; and determine that the user is: exercising below the anaerobic threshold if the velocity growth factor is less than the anaerobic velocity growth factor or less than the lower limit of the anaerobic velocity growth factor range; or exercising at the anaerobic threshold if the velocity growth factor is equal to the anaerobic velocity growth factor or within the anaerobic velocity growth factor range; or exercising above the anaerobic threshold if the velocity growth factor is greater than the anaerobic velocity growth factor or greater than the upper limit of the anaerobic velocity growth factor range.

The ultrasound transducer may be further configured to: send the series of ultrasound waves to a blood flow in the body before exercise begins at a first frequency; and send the series of ultrasound waves to a blood flow in the body during exercise at a second frequency.

The processor may be further configured to calculate the velocity growth factor further based on the first and second frequencies and the doppler angle.

The device may further comprise a button configured to send a signal to the processor that exercise has started when the button has been pressed by the user.

The device may further comprise an attachment means configured to hold the ultrasound transducer attached to the body of the user in use.

The device may further comprise one or more of the following: clock(s) and/or oscillator(s) and/or digital to analog converter(s) (DAC(s)) and/or transmitter(s) and/or receiver(s) and/or transmit-receive switche(s) and/or amplifier(s) and/or analog to digital converter(s) (ADC(s)) and/or filter(s) and/or memory(ies) and/or power source(s).

The device may provide the advantage of determining when the user is exercising below, at or above the anaerobic threshold without requiring blood sampling or breath collection.

According to a second aspect of the invention, there is provided a method of determining when a user is exercising below, at or above the anaerobic threshold, the method comprising the steps of:
a) providing a device according to the first aspect of the invention;
b) attaching the ultrasound transducer to the body of the user before the user begins exercising;
c) sending a first series of ultrasound waves at a doppler angle of between 0 and 80 degrees to a blood flow in the body while the user is not exercising;
d) receiving a first series of ultrasound wave reflections back from the blood flow with a doppler shift; and
e) sending a second series of ultrasound waves at the same doppler angle of between 0 and 80 degrees to a blood flow in the body during exercise;
f) receiving a second series of ultrasound wave reflections back from the blood flow with a doppler shift; and
g) sending the first ultrasound wave data to the processor;
h) sending the second ultrasound wave data to the processor;
i) determining, at a first period in the cardiac phase, the doppler shift of the first series of waves;
j) determining, at a second period corresponding to the first period in the cardiac phase, the doppler shift of the second series of waves;
k) calculating a velocity growth factor based on the doppler shifts at the first and second corresponding periods in the cardiac phases;
l) comparing the velocity growth factor with a predetermined anaerobic velocity growth factor or a predetermined anaerobic velocity growth factor range; and
m) determining that the user is:
   i. exercising below the anaerobic threshold if the velocity growth factor is less than the anaerobic velocity growth factor or less than the lower limit of the anaerobic velocity growth factor range; or
   ii. exercising at the anaerobic threshold if the velocity growth factor is equal to the anaerobic velocity growth factor or within the anaerobic velocity growth factor range; or
   iii. exercising above the anaerobic threshold if the velocity growth factor is greater than the anaerobic velocity growth factor or greater than the upper limit of the anaerobic velocity growth factor range.

The step of sending a first series of ultrasound waves may further comprise sending the first series of ultrasound waves at a first frequency.

The step of sending a second series of ultrasound waves may further comprise sending the second series of ultrasound waves at a second frequency.

The step of calculating the velocity growth factor may be further based on the first and second frequencies and the doppler angle.

The method may further comprise the step of: d1) the processor detecting that exercise has started; wherein step d1 is performed between steps d) and e).

The step of the processor detecting that exercise has started may comprise the user providing an input to the processor to indicate that exercise has started.

The method may be such that either: step g) is performed before step e); or step g) and step i) are both performed before step e).

The method may further comprise, after step m), repeating steps e), f), h), j), k), I) and m) again.

The method may provide the advantage of determining when the user is exercising below, at or above the anaerobic threshold without requiring blood sampling or breath collection.

According to a third aspect of the invention, there is provided a device for determining the anaerobic threshold of a user, the device comprising an ultrasound transducer and a processor wherein: the ultrasound transducer is signal connected to the processor and the ultrasound transducer is configured in use to: be attached to the body of the user; send a first, a second and a third series of ultrasound waves at the same doppler angle of between 0 and 80 degrees to a blood flow, wherein the first, second and third series of ultrasound waves can be sent to the body during exercise wherein each series of ultrasound waves can be sent when the body is at different exercise intensities; receive first, second and third series of ultrasound wave reflections back from the blood flow with a doppler shift; and send the ultrasound wave data to the processor; wherein the processor is configured to: receive ultrasound wave data from the ultrasound transducer and: determine, at corresponding periods in the cardiac phase, the doppler shift of each the three series of ultrasound waves sent to the blood flow; calculate a first velocity growth factor based on the doppler shifts of the first and second series of ultrasound waves at corresponding cardiac phases of the series; calculate a second velocity growth factor based on the doppler shifts of the second and third series of ultrasound waves at corresponding cardiac phases of the series; compare the first and second velocity growth factors; and determine the anaerobic threshold of the user as the point where the difference between the first and second velocity growth factors is greater than a predetermined value.

The device may further comprise one or more of the following: clock(s) and/or oscillator(s) and/or digital to analog converter(s) (DAC(s)) and/or transmitter(s) and/or receiver(s) and/or transmit-receive switche(s) and/or amplifier(s) and/or analog to digital converter(s) (ADC(s)) and/or filter(s) and/or memory(ies) and/or power source(s).

The device may allow determination the anaerobic threshold in blood directly, without requiring blood sampling or breath collection.

Additionally, the device may allow a significantly more accurate and reliable measurement than conventional estimations such as using the estimated maximum heart rate.

According a fourth aspect of the invention, there is provided a method of determining the anaerobic threshold of a user, comprising the steps of:
a) providing a device according to the third aspect of the invention;
b) attaching the device to the body of a user;
c) sending a first, a second and a third series of ultrasound waves at the same doppler angle of between 0 and 80 degrees to a blood flow in the body during exercise, wherein each series of ultrasound waves is sent at different exercise intensities;
d) receiving a first, a second and a third series of ultrasound wave reflections back from the blood flow with a doppler shift; and
e) sending the ultrasound wave data to the processor;
f) determining, at corresponding periods in the cardiac phases, the doppler shift of each of the three series of waves;
g) calculating a first velocity growth factor based on the doppler shifts of the first and second series of ultrasound waves at corresponding periods in the cardiac phases of the series;
h) calculating a second velocity growth factor based on the doppler shift of the second and third series of ultrasound waves at corresponding periods in the cardiac phases of the series;
i) comparing the first and second velocity growth factors; and
j) determining the anaerobic threshold of the user as the point where the difference between the velocity growth factors is greater than a predetermined value.

The method may further comprise, after step i) and before step j): determining that the difference between the first and second velocity growth factors is not greater than a predetermined value, sending further series of ultrasound waves at the same doppler angle of between 0 and 80 degrees to a blood flow in the body during exercise and different exercise intensities from the previous series of ultrasound waves; receiving further series of ultrasound wave reflections back from the blood flow with a doppler shift; and sending the ultrasound wave data to the processor; determining, at corresponding periods in the cardiac phases, the doppler shift of each of the further series of waves; calculating further velocity growth factors based on the doppler shifts at corresponding periods in the cardiac phases of the further series of ultrasound waves; and comparing the velocity growth factors.

The method may provide the advantage of determining the anaerobic threshold in blood directly, without requiring blood sampling or breath collection.

Additionally, the method may provide a significantly more accurate and reliable measurement than conventional estimations such as using the estimated maximum heart rate.

### BRIEF DESCRIPTION OF THE DRAWINGS

The prior art and embodiments of the present disclosure will now be described, by way of example only, with reference to the accompanying drawings, in which:
Fig. 1 shows an illustration of the concentration of lactate in blood vs running speed on an example human;
Fig. 2 shows an illustration gas concentration of oxygen and carbon dioxide concentration during a test;
Fig. 3 shows a device according to the present invention attached to the wrist of a user;
Figs. 4 and 5 show front and rear view of the device shown in Fig. 3;
Fig. 6 shows an illustration of exercise intensity vs blood flow velocity;
Fig. 6a shows an illustration of exercise intensity vs blood flow velocity, with possible sampling points along the exercise intensity X axis marked;
Figs. 7, 8 and 9 show schematic drawings of the ultrasound transducer of the device in Fig. 3 in use;
Fig. 10 shows ultrasound transducer of the device in Fig. 3 arranged at an angle to provide the ultrasound beam at an angle;
Fig. 11 shows an alternative sliced ultrasound transducer arrange to provide the ultrasound beam at an angle;
Fig. 12 shows an alternative ultrasound transducer provided with a lens to provide acoustic steering of the ultrasound beam;
Fig. 13 shows the device's wireless communication with a fitness watch;
Fig. 14 shows a flow diagram of a method to determine whether a user is exercising below, at or above the anaerobic threshold; and
Figs. 15 and 16 show flow diagrams relating to a method to determine the anaerobic threshold of a user.

### DETAILED DESCRIPTION

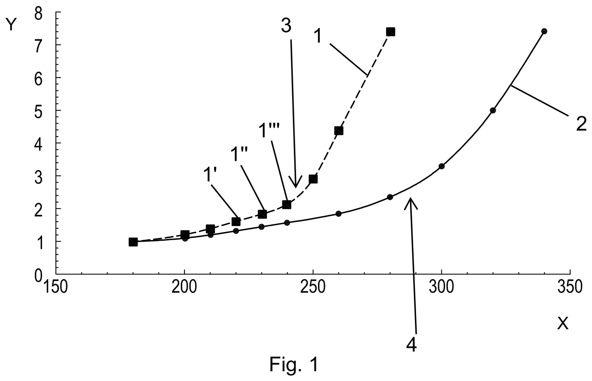

Fig. 1 shows an illustration of the concentration of lactate in blood (mM) on the Y-axis and the running speed (m.min⁻¹) of an example human on the X-axis. In the illustration, the running speed vs lactate concentration of a poor runner is shown by the dashed line 1 and the running speed vs lactate concentration of a good runner is shown by the solid line 2. According to prior art methods, the lactate concentration is measured at a series of points 1', 1", 1‴ etc. with the trends interpolated between the data points for both the good runner and poor runner.

As the running speed increases, a level is reached (around points 3 and 4 for the poor runner and good runner respectively) where the blood lactate is quickly increased, the reason for which has been previously explained. The problem, in terms of athletic performance, with having too much lactate in the muscles is that the muscles become sore and it takes a longer time to recover for the next exercise. As shown in the illustration, a good runner can run faster before the blood lactate increases, but in the end even good runners will experience too much lactate in the muscles. Typically above 3mM to 4mM is considered as excessive lactate.

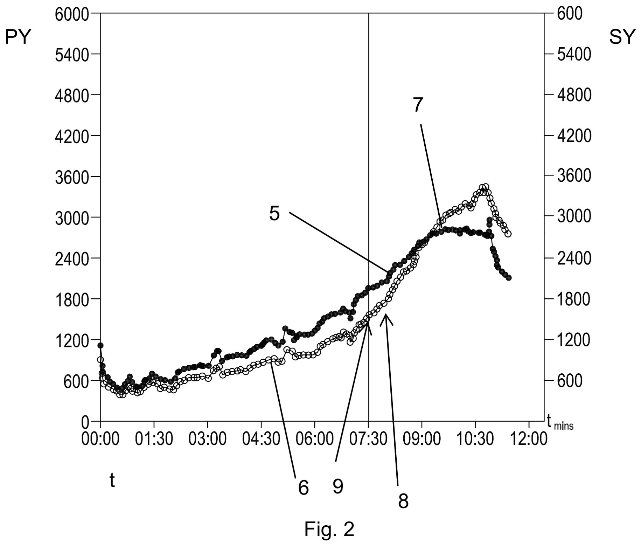

Fig. 2 shows an illustration of a prior art test to determine the anaerobic threshold wherein the gas concentration of oxygen and carbon dioxide concentration in the exhalation air of an athlete are measured during a test exercise session. In the illustration oxygen concentration ml/min is plotted against the primary Y-axis (PY) and indicated as line 5 in the illustration.

Carbon dioxide concentration ml/min is plotted against the secondary Y-axis (SY) and is indicated as line 6 in the illustration. Again, the illustration is formed of a series of data points measured during test, with interpolation between the data points providing the trend for both oxygen concentration and carbon dioxide concentration.

The elapsed time during the test is plotted on the X-axis (t). Typically in such a test, the exercise intensity is increased every minute until exhaustion of the athlete. Exhaustion can be seen in the illustration at point 7 where the oxygen concentration 5 does not continue to increase as exercise intensity increases. At an earlier point 8 in the test exercise session, the concentration of carbon dioxide 6 increases faster than the concentration of oxygen 5, as can clearly be seen by the converging plots on the illustration at point 8. The point 9 where the concentration of carbon dioxide 6 starts to increase faster than the concentration of oxygen 5 is the anaerobic threshold. As explained previously, the anaerobic threshold 9 and lactate threshold cannot be measured in the same way, but it is generally accepted that the anaerobic threshold 9 and lactate threshold occur at the same time or around the same time. Again, for the purposes of guiding training, most athletes accept that the anaerobic threshold and lactate threshold are the same or almost the same for this purpose.

Oxygen measurements require a mask during the athletic activity, such as running, therefore this technique is less in use by athletes during training, and it is instead mainly used for research purposes.

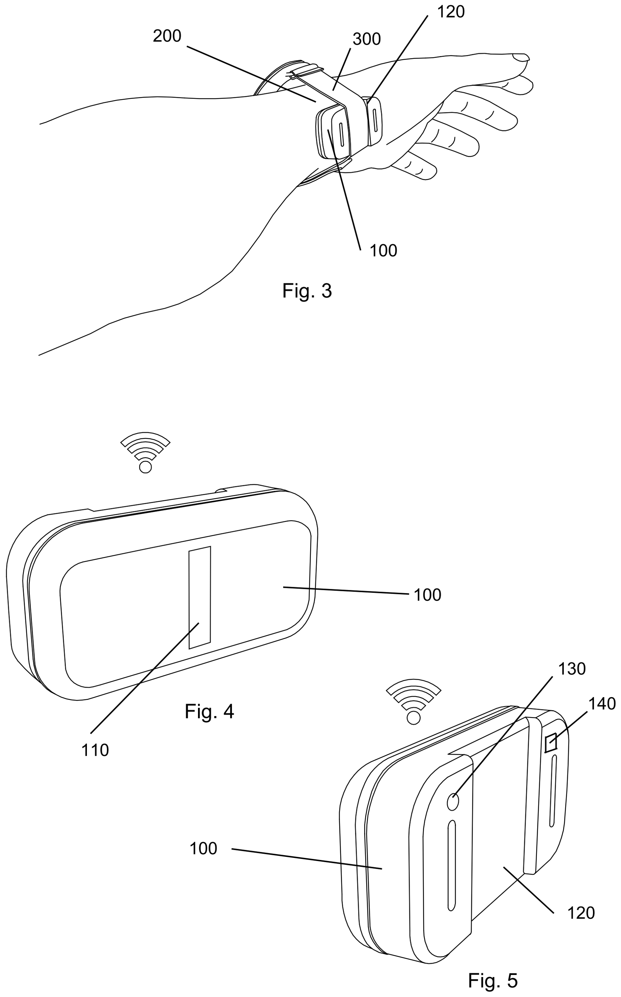

Fig. 3 shows a device 100 in accordance with a first embodiment of the present disclosure. The device 100 is for measuring doppler shifts of a blood flow inside the human body before and during exercise, and using these measurements to calculate the velocity growth factor.

In a second embodiment of the present disclosure, the device 100 is for measuring doppler shifts of a blood flow inside the human body before and during exercise, and using these measurements, the ultrasound frequencies and the estimated doppler angle to calculate the velocity growth factor.

In a third embodiment of the present disclosure, the device 100 is for measuring doppler shifts of a blood flow inside the human body during exercise, and using these measurements to determine the anaerobic threshold of the user.

As shown in Fig. 3 the device 100 is attached in use to the wrist 200 of a user. It will be understood that the wrist 200 of a user is a particularly suitable area for measurement of doppler shift of blood flow due to the proximity to major arteries. More specifically, the radial artery and ulnar artery are in proximity to the wrist 200.

The wrist 200 is an ideal location for measuring doppler shift of the blood flow due to the minimal obstruction by muscle or fat. Although professional athletes may utilise monitors in obscure locations on the body without issue, amateur athletes or casual exercisers will typically prefer monitors located at common locations such as the wrist 200. The wrist 200 is usually accessible to the user before and during exercise and devices placed on the wrist 200 can be easily adjusted and taken on/off before and during exercise. Furthermore, the user may already be used to wearing a watch or other monitoring devices on their wrist 200.

Figs. 4 and 5 show the device 100 off of the wrist 200 of the user. As can be seen in Fig. 4, the device 100 comprises an ultrasound transducer 110 and a groove 120 located on the opposite side of the device 100 from the ultrasound transducer 110.

To be able to use the device 100 to measure doppler shift of the blood flow during exercise, as is required in the first, second and third embodiments of the present disclosure, it is important that the device 100 is temporarily fixed to the wrist 200 and thus following the motion of the surrounding tissue. In this regard, it is said that the device 100 is attached to the wrist 200 of a user. Although it is preferable that the device 100 is attached directly to the wrist 200 without any intermediate layers, it will be understood that attached to the wrist 200 may also cover attached with an intermediate layer, which does not affect the ultrasound waves, located between the device 100 and the wrist 200.

If the ultrasound transducer 110 is too large or held by hand, motion artefacts will be introduced affecting the measurements.

The ultrasound transducer 110 in the presently described example is a spectral ultrasound doppler, however it will be understood that in other examples other ultrasound devices may be used. Only the major components of the device 100 are described in the present disclosure in the interest of clarity and brevity. In this connection, it will be understood that the device 100 may further comprise one or more of the following components: clock(s) and/or oscillator(s) and/or digital to analog converter(s) (DAC(s)) and/or transmitter(s) and/or receiver(s) and/or transmit-receive switche(s) and/or amplifier(s) and/or analog to digital converter(s) (ADC(s)) and/or filter(s) and/or memory(ies) and/or power source(s).

It will also be appreciated that an ultrasound transducer 110 utilising pulsed wave or continuous wave doppler, or both, may be implemented in some examples. In this connection, although continuous wave may be utilised, pulsed wave is preferable due to the possibility to automatically extract areas above or below the artery and measure the tissue velocities and compensate for these in the blood flow velocity estimation.

As can be seen in Fig. 3, the groove 120 is for attaching a band 300 to secure the device 100 to the wrist 200 of the user. The band 300 may be made of an elastic fabric. It will be understood that there are many other ways of attaching the device 100 to the wrist 200 of the user. The presently described example of utilising a band 300 and groove 120 to locate and hold the band 300 within is just one example. In some examples the band 300 may be permanently attached to the device 200 rather than being a separate object. In some examples, the device 100 may be incorporated within another device such as a fitness monitor, a smart watch, a fitness tracker or a similar device. In some examples, the ultrasound transducer 110 may be incorporated within another device such as a smart watch, fitness watch or fitness tracker. In this regard, the device 100 may actually be a smart watch, fitness watch or fitness tracker with other functions in addition to hosting the ultrasound transducer 110 for the purposes of the present invention.

As in the presently described example, the device 100 may be wirelessly connectable to another device such as a fitness watch, mobile phone, laptop computer, tracker or screen, thus allowing the user or their coach to see real time information relating to the training zone in which they are currently exercising within. In some examples the device 100 may be connected by wired means to the display device. In some examples, the device 100 may comprise a screen configured to display information to the user.

The device 100 according to the first embodiment of the disclosure, i.e. the device 100 configured for measuring doppler shifts of a blood flow inside the human body before and during exercise, and using these measurement to calculate the velocity growth factor, is now explained in more detail.

In use, the ultrasound transducer 110 measures the doppler shift of the blood flow by emitting an ultrasound wave into the wrist 200 of the user. Typically the ultrasound wave will be a high-frequency ultrasound wave.

The waves travel through the body and when the waves encounter a blood flow the waves bounce back to the ultrasound transducer 110. Doppler effect occurs when the frequency of the waves change as they are reflected off of the moving red blood cells in the blood flow. When blood is moving towards the ultrasound transducer 110, the frequency of the received waves increases. When blood is moving away from the ultrasound transducer 110, the frequency of the received waves decreases. In other words, the ultrasound transducer 110 receives the ultrasound waves reflected back with an adjusted frequency, this being the doppler shift Δf.

The doppler shift Δf equation can be presented as: $Δf = \frac{2 ∗ v ∗ f 0 ∗ cos \left(θ\right)}{c}$ wherein:
Δf is the doppler shift
v is the blood velocity
f0 is the frequency of the transmitted ultrasound wave
c is the speed of sound in tissueΘ is the angle between the vessel and the ultrasound beam

Rearranging the formula for the absolute blood velocity, v: $v = \frac{Δf ∗ c}{2 ∗ f 0 ∗ cos \left(θ\right)}$

If two readings are taken, v₁, v₂: ${v}_{1} = \frac{{Δf}_{1} ∗ c}{2 ∗ {f 0}_{1} ∗ cos \left(θ\right)}$ ${v}_{2} = \frac{{Δf}_{2} ∗ c}{2 ∗ {f 0}_{2} ∗ cos \left(θ\right)}$

The velocity growth factor, the multiple by which a later absolute velocity of the blood flow has increased relative to the originally measured absolute velocity of the blood flow when the user was at rest, is: $Velocity growth factor = \frac{{v}_{2}}{{v}_{1}} = \frac{\frac{{Δf}_{2} ∗ c}{2 ∗ {f 0}_{2} ∗ cos \left(θ\right)}}{\frac{{Δf}_{1} ∗ c}{2 ∗ {f 0}_{1} ∗ cos \left(θ\right)}}$

Simplifying by cancelling out common terms (c, 2, and cos (θ)): $Velocity growth factor = \frac{{Δf}_{2} ∗ {f 0}_{1}}{{Δf}_{1} ∗ {f 0}_{2}}$

It can therefore also be said that, when all other variables of the doppler shift equation are constant, the velocity growth factor also means the ratio of the doppler shift recorded at a later point in time multiplied by the ultrasound frequency at the earlier point in time, when the user was at rest, relative to the doppler shift recorded at an earlier point in time when the user was at rest multiplied by the ultrasound frequency at the later point in time.

In this way, the velocity growth factor of v can be calculated without knowing the angle θ.

In the first embodiment of the present disclosure, the frequency of the ultrasound transducer 110 is maintained constant, such that to determine the velocity growth factor, all that is needed to determine the velocity growth factor is two measurements of the doppler shift, a first Δf₁ taken when the user is at rest and a second Δf₂ taken during exercise, as shown below: $Velocity growth factor = \frac{{Δf}_{2}}{{Δf}_{1}}$

It can therefore also be said that, when all other variables of the doppler shift equation are constant, the velocity growth factor also means the ratio of the doppler shift recorded at a later point in time relative to the doppler shift recorded at an earlier point in time when the user was at rest.

An example calculation is now provided to illustrate how the device 10, configured according to the first embodiment of the disclosure, can calculate the velocity growth factor v, only by measuring two doppler shifts: $Velocity growth factor of v = \frac{{Δf}_{2}}{{Δf}_{1}} = \frac{2.5 kHz}{1 kHz} = 2.5$

Example calculations now provided to illustrate how the device 100 configured according to the second embodiment of the disclosure can calculate the velocity growth factor v, by measuring the two doppler shifts, using the transmit frequencies of the ultrasound waves and an estimated doppler angle for the ultrasound waves.

In a first example calculation below, illustrating a first possible calculation performed by the processor when the device 100 is configured according to the second embodiment of the present disclosure, the following parameters are used:
First frequency of the transmitted ultrasound wave: 5MHz
Second frequency of the transmitted ultrasound wave: 10MHz
First estimated doppler angle of ultrasound waves: 45 degrees
Second estimated doppler angle of ultrasound waves: 45 degrees
First doppler shift reading: 1kHz
Second doppler shift reading: 3kHz
The speed of sound in tissue: 1540 m/s
The velocity growth factor is then calculated as follows: $Velocity growth factor = \frac{{v}_{2}}{{v}_{1}} = \frac{\frac{{Δf}_{2} ∗ c}{2 ∗ {fO}_{2} ∗ cos \left(θ\right)}}{\frac{{Δf}_{1} ∗ c}{2 ∗ {fO}_{1} ∗ cos \left(θ\right)}} = \frac{\frac{3 KHz ∗ 1540 m / s}{2 ∗ 10 MHz ∗ cos \left(45\right)}}{\frac{1 kHz ∗ 1540 m / s}{2 ∗ 5 MHz ∗ cos \left(45\right)}} = 1.5$

In a second example calculation below, illustrating a second possible calculation performed by the processor when the device 100 is configured according to the second embodiment of the present disclosure, the following parameters are used:
First frequency of the transmitted ultrasound wave: 5MHz
Second frequency of the transmitted ultrasound wave: 5MHz
First estimated doppler angle of ultrasound waves: 45 degrees
Second estimated doppler angle of ultrasound waves: 45 degrees
First doppler shift reading: 1kHz
Second doppler shift reading: 3kHz
The speed of sound in tissue: 1540 m/s
The velocity growth factor is then calculated as follows: $Velocity growth factor = \frac{{v}_{2}}{{v}_{1}} = \frac{\frac{{Δf}_{2} ∗ c}{2 ∗ {fO}_{2} ∗ cos \left(θ\right)}}{\frac{{Δf}_{1} ∗ c}{2 ∗ {fO}_{1} ∗ cos \left(θ\right)}} = \frac{\frac{3 KHz ∗ 1540 m / s}{2 ∗ 5 MHz ∗ cos \left(45\right)}}{\frac{1 kHz ∗ 1540 m / s}{2 ∗ 5 MHz ∗ cos \left(45\right)}} = 3$

In the second embodiment of the present disclosure, the doppler angle of the ultrasound beam may contain an error. This does not affect the calculation of the velocity growth factor, since the error is cancelled out in the calculation. Using the device 100 configured according to the second embodiment of the present disclosure, the device 100 can be said to calculate the absolute velocities first and then calculate the velocity growth factor from the absolute velocities. It again does not matter that the absolute velocities may have an error factor in their values, as they are both subject to the same error which cancels out, therefore so long as the absolute velocities are used for the calculation of velocity growth factor, the presence of this error is not important.

Said another way, the device 100 is configured in the first embodiment of the disclosure to calculate velocity growth factor based on measurement only of the doppler shift.

The device 100 is configured in the second embodiment of the present disclosure to calculate velocity growth factor based on absolute velocities calculated from measurement of the doppler shift, approximation of the doppler angle of the ultrasound waves and the known transmit frequencies of the ultrasound waves. As highlighted above, in some examples according to the second embodiment of the present disclosure, the first and second frequencies may be the same.

Regardless of configuration of the device 100 to calculate the velocity growth factor, the device 100 according to the first and second embodiments of the present disclosure are configured to perform a first doppler shift reading Δf₁ when the user is at rest.

The second doppler shift reading Δf₂ is taken when the user is exercising and, as above, the velocity growth factor is calculated based on the ratio of the second doppler shift reading Δf₂ with respect to the first doppler shift reading Δf₁ in the first embodiment of the present disclosure, and on this and further parameters in the second embodiment of the present disclosure, as described.

The device 100 will perform the above calculation repeatedly to calculate multiple velocity growth factors corresponding to multiple successive doppler shift readings Δf₂ which are successively compared with the doppler shift reading Δf₁ obtained when the user was resting.

In both the first and second embodiments of the present disclosure, consistency in selecting the reference point within the pulse wave is essential for accurate and comparable measurements of doppler shift across the series. As previously explained, measurement of the arterial blood flow may be peak systolic doppler shift or end diastolic doppler shift or time averaged doppler shift. If two different pulses are used for measurement, as is the case in the first and second embodiments of the present disclosure, the same period must be used to ensure a valid comparison. If utilising peak systolic doppler shift or end diastolic doppler shift measurements, the period may be very small, such that it may appear as a single point. When utilising time averaged doppler shift, the period may be longer.

Although peak systolic doppler shift, end diastolic doppler shift and time averaged doppler shift are mentioned, it will be understood that measurement markers featuring any characteristic feature that remains consistent across pulses may be used in alternative examples not described herein.

On the contrary, measuring different periods in each pulse could introduce discrepancies, as different phases may exhibit varying frequency shifts.

The inventor has discovered that for the average person the anaerobic threshold is when the blood flow has a velocity growth factor of around four with respect to the resting blood flow velocity. That is to say, the anaerobic threshold is when the blood flow velocity is around four times the resting blood flow velocity.

Since the anaerobic threshold is not a narrowly defined point, it is reasonable that the anaerobic threshold is considered to be an interval around the point where the blood flow velocity is four times the resting blood flow velocity. In this connection, the device may be configured to determine that the user is below the anaerobic threshold when the growth factor is below three and three quarters, is at the anaerobic threshold when the growth factor is between three and three quarters and four and a quarter and is above the anaerobic threshold when the growth factor is above four and a quarter. In alternative arrangements, the anaerobic threshold range may be set more broadly or narrowly.

The arterial blood flow is pulsatile. The processor may be set up to automatically identify various phases of the cardiac cycle from the ultrasound pulses, like peak-systole and end-diastole. When calculating the velocity growth factor, it is required to use doppler shifts corresponding to the same period of the cardiac phase in the two series.

Measurement of the arterial blood flow may be peak systolic doppler shift or end diastolic doppler shift or time averaged doppler shift. It may be possible to utilise any of these measurements or other measurements in the invention. Time averaged doppler shift over one heartbeat (end-diastole to end-diastole) is the preferred measurement since it is most robust. In some examples, additional robustness may be added by averaging over multiple heartbeats. In some examples clutter filtering may be used to remove motion artefacts from surrounding tissue for additional robustness but this is not essential.

Although not essential for the implementation of the invention, it is preferred to automatically detect and select the sample volume at the depths where the artery is located, to increase the sensitivity and the signal to noise ratio of the signal from the artery. Automatic selection of the sample volume can be performed based on selecting depths where the velocity is above a certain threshold, at which tissue motion is unlikely.

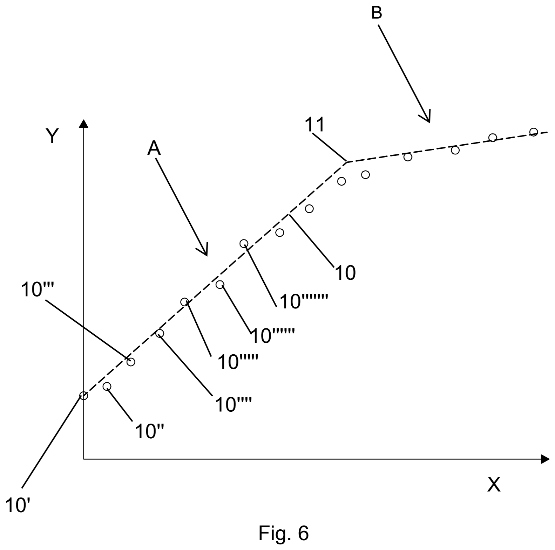

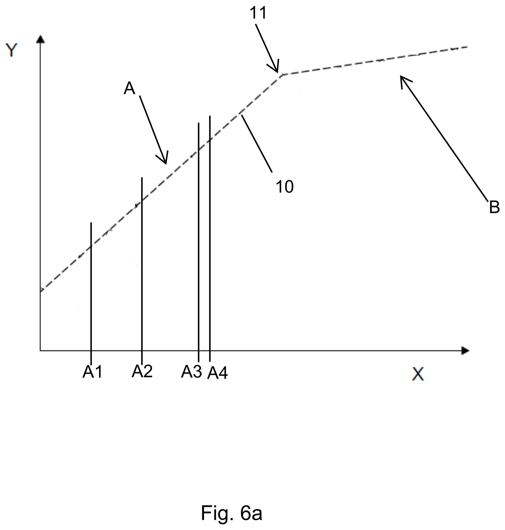

Fig. 6 shows an illustration of exercise intensity (in Watts) on the X-axis against blood flow velocity (in ml/min) on the Y-axis. The illustration is formed of a series of data points 10', 10", 10"', 10ʺʺ etc. where the blood flow velocity is plotted at a corresponding exercise intensity. Interpolation of the data points 10', 10", 10‴ then provides the blow flow velocity against exercise intensity trend 10. It can be seen that the blood flow velocity increases linearly in section A of the illustration as the exercise intensity is increasing. When exercising is started the muscles recruit many more capillaries and the arterials dilate. When these effects reach their maximum, towards the upper end of section A, the amount of blood being provided to the muscles becomes limited, therefore the amount of oxygen supplied to the muscles begins to flatten out.

Referring still to the illustration in Fig. 6 it can be seen that in section A the blood flow velocity increases linearly until the anaerobic threshold 11 is met. At the anaerobic threshold 11, the blood will no longer be able to provide enough oxygen or transport the additional created lactate away from the muscles, and the lactate levels increase rapidly. At this point, there is a marked change in the rate of change of blood flow velocity as the exercise intensity increases by a fixed amount. Attention is drawn to the rate of change of blood flow velocity in section B in the illustration in Fig. 6, which is above the anaerobic threshold 11, compared with section A which is below the anaerobic threshold 11.

The device 100 in use according to the first and second embodiments of the present disclosure, takes a reading of the doppler shift at rest, before exercise begins.

Regardless of whether the device 100 is configured according to the first or second embodiment of the present disclosure, the device 100 may be configured in a plurality of ways to allow the user to take this initial reading when the user is at rest. In some examples, the device 100 may comprise a button, such as push button 130 shown in Fig. 4. In such cases, the user may push the push button 130 to indicate they are at rest such that the device 100 can take a first doppler shift reading as previously discussed.

In some examples, the device 100 may be taking doppler shift readings continuously and storing the doppler shift data in its memory. In such cases, the user may push the push button 130 to indicate that exercise has started, such as directly before starting cycling, running, swimming or any other form of exercise. The device 100 may then retrieve the resting doppler shift reading from 5 seconds or 10 seconds or 15 seconds before the push button 130 was pressed, for example. It will be understood by a person skilled in the art that the specific point in time at which the device 100 records the resting doppler shift will depend on the instruction given to the user, for example if the user is instructed to push the push button 130 when they are completely at rest and not beginning to exercise at all, then the device 100 may take that moment in time as the resting doppler shift. Where the device 100 goes back in time to take the resting doppler shift value, it will be understood that it may apply some statistical analysis to the readings to ensure that the user was not actually exercising.

In some examples, the device 100 may not comprise a push button 130 or similar for the user indicating when the user is about to start exercising. Instead, the device 100 may be configured to detect that the user has started exercising. One possible way that the device 100 may detect that the user has started exercising is that there is a marked change in the doppler shift readings. Alternatively, the device may be equipped with other sensors to detect that the user has started exercising.

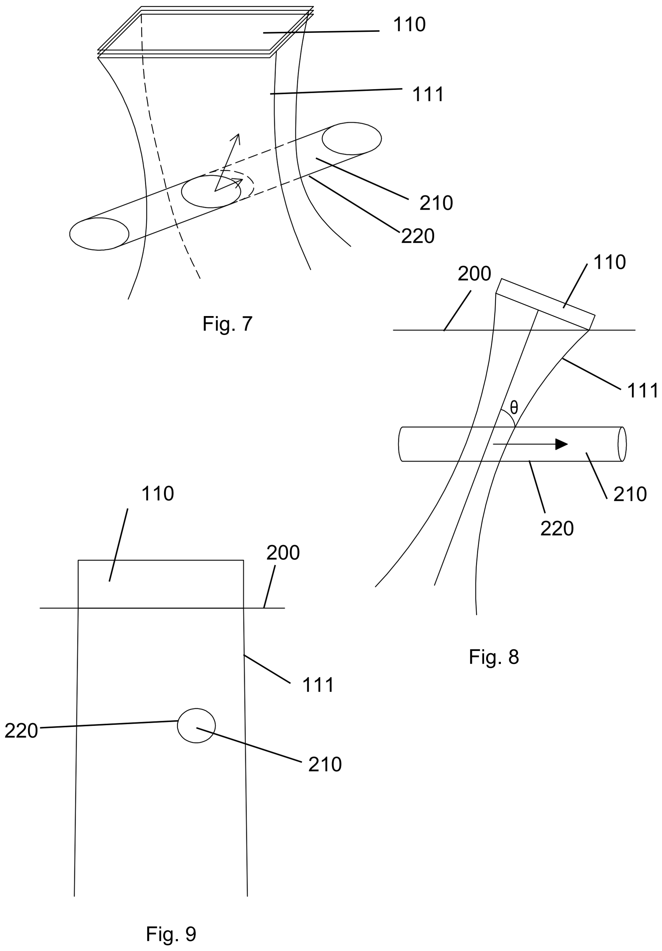

Figs. 7, 8 and 9 show schematic diagrams of the ultrasound transducer 110 and the associated ultrasound beam 111 into the wrist 200. In the presently described examples, the ultrasound transducer 110 is a rectangular transducer which is unfocused in one dimension and slightly focused in the other. A rectangular transducer is more robust for use with a person in motion. In this connection, with a rectangular transducer, the artery may be kept in the ultrasound field more easily.

Referring to Fig. 7, the ultrasound beam 111 is sent into the wrist 200 and reaches the blood flow 210 in the artery 220. Fig. 8 provides a lateral view of the wrist 200.

In order to measure the doppler shift of the blood flow 210, the angle Θ between the artery and the ultrasound beam must be between 0 and 80 degrees. This angle between the artery and the ultrasound beam is often referred to as the doppler angle Θ. It is preferable, although not essential, to provide a doppler angle of between 0 and 60 degrees. A doppler angle Θ of between 0 and 60 degrees may provide a more accurate result.

The invention according to the first embodiment of the present disclosure does not require the specific angle Θ between the ultrasound beam and the artery to be known or measured, as long as this angle Θ is maintained constant across readings, due to the cancellation of the Θ in the equation when calculating the velocity growth factor.

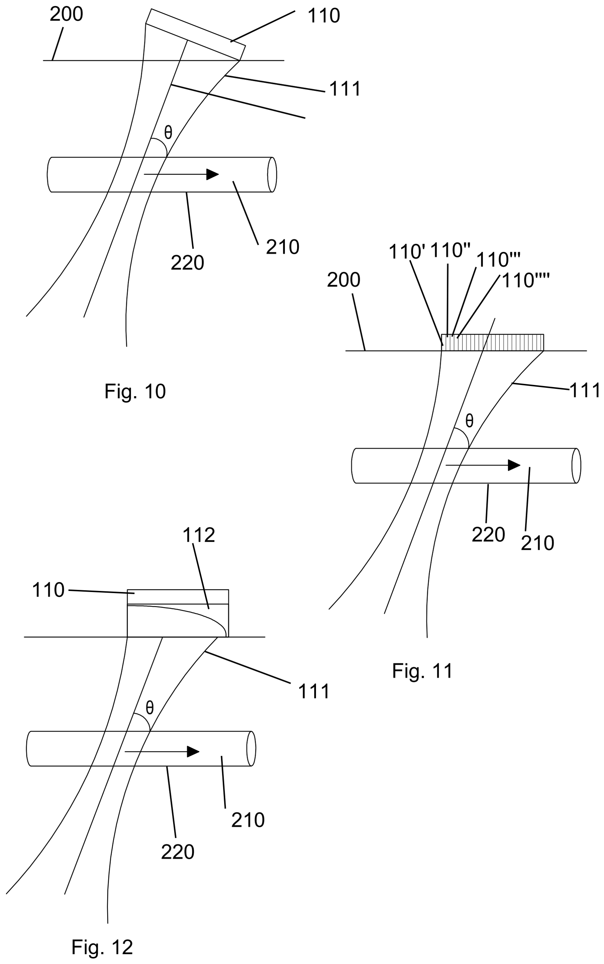

Three possible ways of providing the ultrasound beam 111 at the required angle Θ are now shown schematically in Figs. 10, 11 and 12. In order to provide the ultrasound beam at the required angle Θ of between 0 and 80 degrees, the ultrasound transducer 110 may simply be tilted as shown in Fig. 10. In order to provide the ultrasound beam at the preferred angle Θ of between 0 and 60 degrees, the ultrasound transducer 110 may simply be tilted as shown in Fig. 10. The angle of tilt may be fixed within the device 100 (not shown in Fig. 10) holding the ultrasound transducer 110.

In an alternative arrangement shown in Fig. 11, the ultrasound transducer 110 is sliced in many elements 110', 110", 110"', 110ʺʺ, with a delay applied to each transducer element, thereby "steering" the ultrasound beam 111. In such cases, the sliced transducer is substantially flat on the wrist 200 of the user, but the provided ultrasound beam 111 is at an angle Θ as shown in Fig. 11.

In an alternative arrangement shown in Fig. 12, the ultrasound transducer 110 is provided with a lens 112, thereby acoustically "steering" the ultrasound beam 111. In such cases, the acoustically steered ultrasound beam 111 is provided by an ultrasound transducer 110 arranged substantially flat on the wrist 200 of the user, but the provided ultrasound beam 111 is at an angle Θ as shown in Fig. 12.

Regardless of the arrangement provided, the specific angle Θ does not need to be known so long as the ultrasound beam 111 is maintained at the same angle across all measurements.

In a use of the device 100 wherein the user measures the same artery, at the same location and at the same angle Θ, the specific Θ is not required as the angle is cancelled out when the velocity growth factor of the blood flow is calculated.

Optionally, the heart rate from the ultrasound signal may be determined and provided to user. This may provide useful additional information from the ultrasound measurement if the user is continuing to increase intensity even beyond the anaerobic threshold.

For the avoidance of doubt, the heart rate measurement is not required to monitor if the user is below, at or around or above the anaerobic threshold, but can be provided to the user and may be particularly interesting to the user when the user is exercising above the anaerobic threshold 11.

If the ultrasound beam angle Θ is known, it will be understood that the blood flow velocities calculated using the ultrasound beam angle Θ may be displayed to the user. However, the ultrasound beam angle Θ is not required to be known to perform the calculation of the velocity growth factor, as previously explained.

In some examples the device 100 may be preprogramed with the velocity growth factor ranges that are determined to be below anaerobic threshold, at anaerobic threshold and above anaerobic threshold. In some examples, the velocity growth factor may be adjustable, for example as better scientific research is performed to determine the average velocity growth factor for a population or a group within a population. In some examples, the device may be preprogramed with multiple sets of velocity growth factors which correspond to different demographics in the population. For example, the user may be able to select their age, weight etc. and be assigned velocity growth factor ranges.

In some examples the device 100 may be provided with a light, for example an LED light or multiple LED lights. The one or more LED lights may be illuminated green when below the anaerobic threshold, amber when at the anaerobic threshold and red when over the anaerobic threshold, for example. Similarly, a single LED light may be illuminated when at the anaerobic threshold, in some examples.

In some examples the device 100 may be configured to provide haptic feedback to the wrist 200 of the user, thereby indicating that the user is exercising at the anaerobic threshold. Alternatively, other forms of tactile feedback may be utilised.

Although in the presently described examples, the ultrasound transducer 110 serves to send and receive ultrasound waves, it will be understood that in some examples an ultrasound transmitter and ultrasound receiver may be provided as separate components.

It will be understood that the device 100 in the presently described example is equipped with a processor 140 to perform the required calculation of the velocity growth factor.

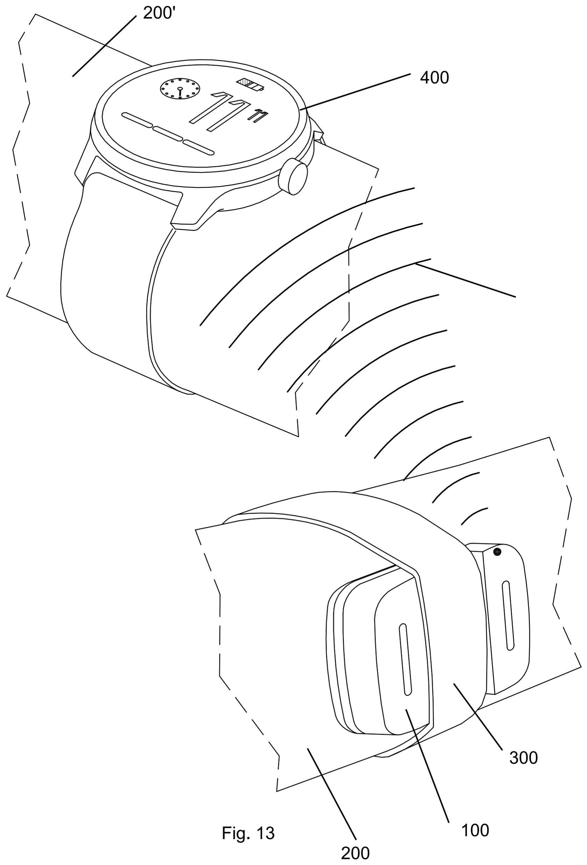

As shown in Fig. 13, in the presently described example, the device 100 is equipped with a wireless communication means to communicate with a fitness watch 400. Again, it will be understood that the other device may instead be a smartwatch, fitness tracker, laptop or any other suitable other device. In the presently described example, the user is wearing the device 100 on one of their wrists 200, and the fitness watch 400 on another of their wrists 200'. It should be noted that the previously discussed button 130 and processor 140 that are shown in Fig. 5 have been omitted from Fig. 13.

The device 100 may be equipped with readable memory such that the processor 140 can store and access instructions from the readable memory regarding the calculations to be performed. Further, the readable memory may be writeable by the processor 140, such that processor 140 can store, temporarily or for a longer period of time, information relating to the recorded doppler shift Δf or, where the angle of the ultrasound beam Θ is known or can be estimated and the transmit frequency of the ultrasound waves is known or is constant, the absolute blood velocities.

In some examples, the device 100 may be connected to a cellular network, for example using 3G, 4G, 5G or LTE technology. In such cases the instructions for the processor 140 may be stored in the cloud or in another remote storage device and be accessible by the device 100 via the cellular network. Likewise, the device 100 may store data in the cloud via the cellular network. The device 100 may be Bluetooth enabled.

In alternative examples, the device 100 may not be equipped with a processor 140, and the processor 140 may be located at another location, such as but not limited to, in a smartphone or smartwatch. The device 100 may simply gather and send the raw ultrasound data to the processor 140 located at a remote location relative to the device 100.

Although it is necessary for there to be an ultrasound transducer 110 at the location where the doppler shift is measured, it will be understood that all other components of the device 100 may be located remotely from the ultrasound transducer 110 so long as a communication means, wired or wireless is provided such that the data can be transferred from the ultrasound transducer 110 to the location where the processor 140 is provided. That is to say, the processor 140 may be separate from the ultrasound transducer 110.

It will be understood that the display may also be remote from the other components of the device. In some examples, the ultrasound transducer 110 may be distant from the processor 140 and the processor 140 may be distant from the display.

Throughout the present disclosure, the device 100 comprising the ultrasound transducer 110 is explained as being connected or connectable to the wrist 200 of the user. For the avoidance of doubt, the device 100 may be connected to other parts of the human body where a doppler shift reading can be obtained. As non-limiting examples, the device 100 may be located at the neck, chest, upper arm, abdomen, groin or lower leg. The device 100 may be attached to alternative parts of the body where blood vessels are accessible and close to the surface, and particularly where large arteries are present.

As described throughout the present description, there are many different arrangements possible involving the separation or combination of components such as the ultrasound transducer, display (where provided), processor 140 etc. It will be understood by a person skilled in the art that the specific arrangement, locations and connections between these components is not important for the performance of the invention. The minimum components required to utilise the invention are a device for determining when a user is at the anaerobic threshold, the device comprising an ultrasound transducer 110 and a processor 140. The ultrasound transducer 110 is signal connected to the processor 140 and the ultrasound transducer 110 is arranged in use to: be attached to the body of the user during exercise; send at a doppler angle of between 0 and 80 degrees successive ultrasound waves to a blood flow in the body; receive successive ultrasound wave reflections back from the blood flow with a doppler shift; and send the ultrasound wave data to the processor 140; wherein the processor 140 is configured to: receive ultrasound wave data from the ultrasound transducer 110 and determine the doppler shift of the ultrasound waves sent and reflected off of the blood flow; from the doppler shift of successive waves, determine the velocity growth factor of the blood flow; and determine the anaerobic threshold as the point when the velocity growth factor changes beyond a tolerance value.

In the present context, velocity growth factor means the multiple by which a later absolute velocity of the blood flow has increased relative to the originally measured absolute velocity of the blood flow when the user was at rest. In the present case, the velocity growth factor also means the ratio of the doppler shift recorded at a later point in time relative to the doppler shift recorded at an earlier point in time when the user was at rest, when the other variables in the doppler shift equation are constant or assumed to be constant.

In a third embodiment of the present disclosure, the device 100 is for measuring doppler shifts of a blood flow inside the human body during exercise, and using these measurements to determine the anaerobic threshold of the user.

The device 100 according to the third embodiment of the present disclosure, i.e. the device 100 configured to determine the anaerobic threshold for the user, is now explained.

The device 100 is arranged and configured to measure multiple doppler shifts of the blood flow in use, as has been previously described. However, when arranged to determine the anaerobic threshold of the user, the device 100 need not measure the resting doppler shift as in the first and second embodiments of the present disclosure previously described.

Fig. 6a shows an illustration of exercise intensity (in Watts) on the X-axis against blood flow velocity (in ml/min) on the Y-axis. It can be seen that the blood flow velocity increases linearly in section A of the illustration as the exercise intensity is increasing. It can be seen that in section A the blood flow velocity increases linearly until the anaerobic threshold 11 is met. At the anaerobic threshold 11, the blood will no longer be able to provide enough oxygen or transport the additional created lactate away from the muscles, and the lactate levels increase rapidly. At this point, there is a marked change in the rate of change of blood flow velocity as the exercise intensity increases by a fixed amount. Attention is drawn to the rate of change of blood flow velocity in section B in the illustration in Fig. 6a, which is above the anaerobic threshold 11, compared with section A which is below the anaerobic threshold 11.

The device 100 configured according to the third embodiment of the present disclosure is configured to measure the doppler shift at different exercise intensities.

For the purposes of illustration, exemplary sample points A1, A2, A3 and A4 are marked on the illustration in Fig. 6a. It may be noted that points A1, A2, A3 and A4 marked on the illustration in Fig. 6a are not spaced at equally increasing exercise intensities, i.e. with equal spacing between each point on the x-axis of the graph. Although in some examples the sample points may be spaced equally, i.e. the sample points correspond to step-wise increases in exercise intensity, this is not essential. It is required that the sample points used in the calculation are at different exercise intensities, but the difference in intensities between adjacent sample points need not be equal. It will be understood by a person skilled in the art that if two sample points were at the same exercise intensity, this would result in no measurement rate of change of velocity.

Where in the first and second embodiments of the present disclosure, the device 100 can take a doppler shift reading at any exercise intensity after exercise has started and compare this reading with the rest reading, in the configuration according to the third embodiment of the present disclosure the invention, the doppler shift measurements used in the calculation are acquired while exercise is underway. It is not required to know the actual exercise intensities, however during test to determine the anaerobic threshold of a user the user may be asked to exercise at gradually increasing intensities until the device determines that the anaerobic threshold has been reached. In this way the anaerobic threshold is determined. It may be preferable, to very accurately determine the anaerobic threshold, to ask the user to increase the exercise intensity in very small increments when it is expected that the user is approaching their anaerobic threshold. Again, it is not required to incrementally increase the exercise intensity for the device to determine the anaerobic threshold, as will become apparent in due course.

Referring again to the illustration in Fig. 6a, it can be seen that the blood flow velocity increases linearly until the anaerobic threshold 11 is met. The velocity growth factor will be constant when the user is below the anaerobic threshold as can be seen by the illustration in Fig. 6.

Said another way, the growth factor is the slope of the trend 10 in Fig. 6, which represents the rate of change of the velocity per unit increase in exercise intensity. Since the trend 10 is linear below the anaerobic threshold, the growth factor is constant below the anaerobic threshold.

The device 10 configured according to the third embodiment of the present disclosure, is configured to determine the velocity growth factor of successive measurements of doppler shift, for example at A1, A2, A3 and A4. The device configured according to the third embodiment of the present disclosure performs the velocity growth factor calculations below: $Velocity growth {factor}_{1} = \frac{{Δf}_{A 2}}{{Δf}_{A 1}}$ $Velocity growth {factor}_{2} = \frac{{Δf}_{A 3}}{{Δf}_{A 2}}$ $Velocity growth {factor}_{3} = \frac{{Δf}_{A 4}}{{Δf}_{A 3}}$

As can be seen from Fig. 6a, the readings of doppler shift do not need to be taken at certain or even equal increases in exercise intensity because velocity growth factor will always be the same when the user is below the anaerobic threshold 11. It is required that the readings of doppler shift are taken at different exercise intensities.

Similarly to the first and second embodiments of the present disclosure, in the third embodiment of the present disclosure, consistency in selecting the reference point within the pulse wave is essential for accurate and comparable measurements of doppler shift across the readings. As previously explained, measurement of the arterial blood flow may be peak systolic doppler shift or end diastolic doppler shift or time averaged doppler shift. If different pulses are used for measurement, as is the case in the presently described third embodiment of the present disclosure, the same period must be used to ensure a valid comparison. If utilising peak systolic doppler shift or end diastolic doppler shift measurements, the period may be very small, such that it may appear as a single point. When utilising time averaged doppler shift, the period may be longer.

Although peak systolic doppler shift, end diastolic doppler shift and time averaged doppler shift are mentioned, it will be understood that measurement markers featuring any characteristic feature that remains consistent across pulses may be used in alternative examples not described herein.

On the contrary, measuring different periods in each pulse could introduce discrepancies, as different phases may exhibit varying frequency shifts.

The device 100 will monitor the result of the calculation of the velocity growth factor, i.e. the values calculated as Velocity growth factor₁, Velocity growth factor₂ and Velocity growth factor₃ and will determine that the anaerobic threshold has been reached when the velocity growth factor is calculated as a value with a significant difference from the previously calculated velocity growth factor or velocity growth factors.

In some examples, a comparison with the previously calculated value will be made. In other examples, multiple previously calculated values may be averaged and the averaged value may be compared against the most recently calculated velocity growth factor.

It will be understood that it is within the ability of the skilled person to apply a reasonable margin of error to the calculations, such that a difference from the previously calculated values, only caused by normal errors in the doppler reading for example, do not cause an incorrect determination that the anaerobic threshold has been met.

Referring again to Fig. 6, the blood flow velocity increases linearly until the anaerobic threshold 11 is met at which point there is a marked change in the rate of change of blood flow velocity. In a use of the device wherein the user measures the same artery, at the same location and at the same angle Θ, the specific Θ is not required as the angle is cancelled out, as previously discussed.

Optionally, the heart rate from the ultrasound signal may be determined and provided to user. This may provide useful additional information from the ultrasound measurement if the user is continuing to increase intensity even after the blood velocity is saturated, i.e. beyond the anaerobic threshold.

For the avoidance of doubt, the heart rate measurement is not required to determine the anaerobic threshold, but can be provided to the user and may be particularly interesting to the user when the user is exercising above the anaerobic threshold 11.

If the ultrasound beam angle Θ and ultrasound frequency are known, it will be understood that the absolute blood flow velocities, i.e. the individual data points 10'. 10", 10‴ in Fig. 6 may be displayed to the user. In this connection, a simple means of communicating that the user has reached the anaerobic threshold may be to simply plot the data points and allow the user to see when the data points begin to stop increasing linearly. However, the device 100 may not always be attached to the user's wrist (or other location on the human body) at the exact same location, thereby providing the ultrasound transducer 110 at the exact same angle Θ every time. Therefore, it is advantageous to provide a method of determining the anaerobic threshold without requiring accurate measurement of the beam angle Θ, as has been thoroughly described.

Without measurement of the beam angle Θ, through the previously explained cancellation of the angle Θ in the calculation, the velocity growth factor may be displayed to the user. In this connection, a means of communicating that the user has reached the anaerobic threshold can be to display to the user when the velocity growth factor is constant and then alert the user when the velocity growth factor is no longer constant.

The user may be presented with information relating to the training zone in which they are currently exercising. For example, the device 100 may monitor the velocity growth factor and alert the user when the velocity growth factor changes, i.e. at point 11 on Fig. 6. In this way, the device 100 still determines the anaerobic threshold for the user. As previously noted, since the velocity growth factor is calculated by a comparison of the doppler shifts which are measurements of a physical property of the human body, i.e. blood flow, the velocity growth factor may not be perfectly stable while the user is in the below anaerobic threshold range - some tolerance may be provided such that the velocity growth factor may change by a small margin and not be determined by the device 100 to be the expected rate of change of velocity growth factor at the anaerobic threshold 11.

Although theoretically the anaerobic threshold 11 is a considered to be a single point i.e. where there is a transition from predominantly aerobic metabolism to predominantly anaerobic metabolism, in practical terms it is often described as occurring within a small range.

Measurement or determination of the anaerobic threshold according to the present invention also leads to the anaerobic threshold being considered to be a small range rather than a single point. Referring to the illustration in Fig. 6, for simplicity the anaerobic threshold 11 is marked as a single point, however it will be understood that due to the above-described tolerance which may need to be exceeded before the device 100 can conclude that the user is at the anaerobic threshold, the anaerobic threshold 11 is for the purposes of the invention, a small range. This also complies with the real-world physiological transition occurring at the anaerobic threshold. That is, the shift from aerobic to anaerobic metabolism is not an abrupt switch but a gradual process, so defining the exact "point" can be challenging.

Referring again to Fig. 2, the exact point 9 where the concentration of carbon dioxide 6 starts to increase faster than the concentration of oxygen 5 is the theoretical anaerobic threshold, however it is very difficult to pinpoint the exact point where this occurs, particularly if the data set is of a lower resolution.

In practice, athletes are used to the presentation of the anaerobic threshold in a small range rather than a single specific point. For example, a particular runner's anaerobic threshold may be considered to be at a pace of between 6 minutes 30 second and 6 minutes 40 seconds per mile, or a particular cyclist's anaerobic threshold may be considered to be between 280 and 300 watts of power output.

In some examples the device 100 may be provided with a light, for example an LED light or multiple LED lights. The one or more LED lights may be illuminated green when below the anaerobic threshold, amber when at the anaerobic threshold and red when over the anaerobic threshold, for example. Similarly, a single LED light may be illuminated when at the anaerobic threshold, in some examples.

In some examples the device 100 may be configured to provide haptic feedback to the wrist 200 of the user, thereby indicating that the user is exercising at the anaerobic threshold. Alternatively, other forms of tactile feedback may be utilised. It is considered that, by alerting the user that they are at the anaerobic threshold, the device 100 determines for the user what their specific anaerobic threshold is. It may be expected in some examples that the user themselves concludes the actual training intensity, for example, which corresponding to their anaerobic threshold, after the device 100 has alerted the user that they have reached their specific anaerobic threshold.

Although in the presently described examples, the ultrasound transducer 110 serves to send and receive ultrasound waves, it will be understood that in some examples an ultrasound transmitter and ultrasound receiver may be provided as separate components.

It will be understood that the device 100 in the presently described example is equipped with a processor 140 to perform the required calculation of the velocity growth factor based on the doppler shift Δf recorded, i.e. the changes in frequency between the sent and reflected-back ultrasound waves.

As shown in Fig. 13, in the presently described example, the device 100 is equipped with a wireless communication means to communicate with a fitness watch 400. Again, it will be understood that the other device may instead be a smartwatch, fitness tracker, laptop or any other suitable other device. In the presently described example, the user is wearing the device 100 according to the present invention on one of their wrists 200, and the fitness watch 400 on another of their wrists 200'.

The device 100 may be equipped with readable memory such that the processor 140 can store and access instructions from the readable memory regarding the calculations to be performed. Further, the readable memory may be writeable by the processor 140, such that processor 140 can store, temporarily or for a longer period of time, information relating to the recorded velocity growth factors or, where the angle of the ultrasound beam Θ is known, the actual blood velocities.

In some examples, the device 100 may be connected to a cellular network, for example using 3G, 4G, 5G or LTE technology. In such cases the instructions for the processor 140 may be stored in the cloud or in another remote storage device and be accessible by the device 100 via the cellular network. Likewise, the device 100 may store data in the cloud via the cellular network. The device 100 may be Bluetooth enabled.

In alternative examples, the device 100 may not be physically equipped with a processor 140 located on the device 100 beside the ultrasound transducer 110, and instead the processor 140 may be located at another location, such as but not limited to, in a smartphone or smartwatch. The physical device 100 on the user's wrist may simply gather and send the raw ultrasound data to the processor 140 located at a remote location relative to the device 100.

Although it is necessary for there to be a device 100 comprising an ultrasound transducer 110 to be at the location where the doppler shift is measured, it will be understood that all other components may be located remotely from the device 100 so long as a communication means, wired or wireless is provided such that the data can be transferred from the device 100 to the location where the processor 140 is provided.

It will be understood that the display may also be remote from the other components of the device. In some examples, the ultrasound transducer 110 may be distant from the processor 140 and the processor 140 may be distant from the display.

Throughout the present disclosure, the device 100 comprising the ultrasound transducer 110 is explained as being connected or connectable to the wrist 200 of the user. For the avoidance of doubt, the device 100 may be connected to other parts of the human body where a blood flow velocity reading can be obtained. As non-limiting examples, the device 100 may be located at the neck, chest, upper arm, abdomen, groin or lower leg. The device 100 may be attached to alternative parts of the body where blood vessels are accessible and close to the surface, and particularly where major arteries are present.

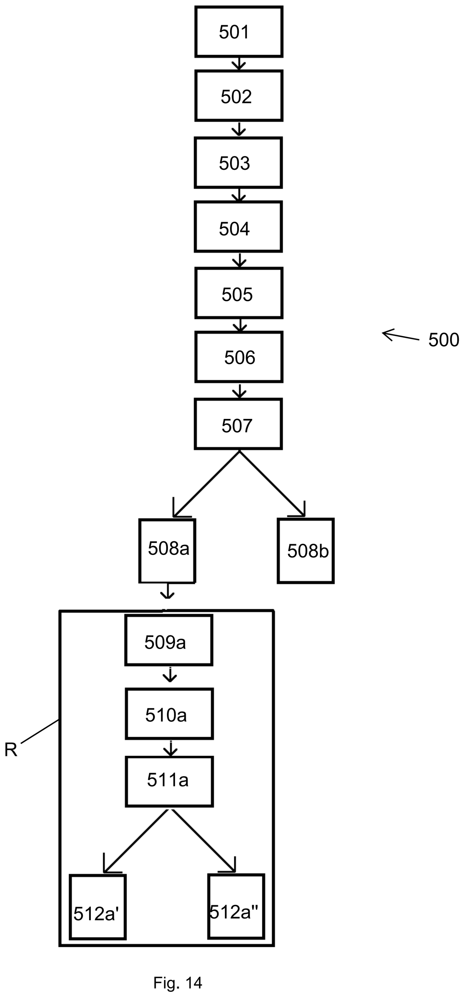

Referring now to Fig. 14, a flow diagram 500 is provided to explain the steps performed, both physical and by the processor 140, to determine when the user is below or at or above the anaerobic threshold using the device 100 according to the first embodiment of the present disclosure.

The flow diagram 500 starts at step 501, wherein the device 100 is attached to the wrist 200 of the user.

At step 502, while the user is resting, i.e. not exercising, the ultrasound transducer 110 sends a first series of ultrasound waves into the wrist 200 of the user at a doppler angle of between 0 and 80 degrees to a blood flow which are reflected, with a first doppler shift, back to the ultrasound transducer 110.

At step 503 the processor 140 determines, at a first period in the cardiac phase, the first doppler shift experienced by the first series of ultrasound waves.

At step 504 the user begins exercising with the device 100 attached to the wrist 200 and the device 100 registers that exercise has begun.

At step 505, the ultrasound transducer 110 sends a second series of ultrasound waves into the wrist 200 of the user at a doppler angle of between 0 and 80 degrees to a blood flow which are reflected, with a second doppler shift, back to the ultrasound transducer 110.

At step 506 the processor 140 determines, at a second period corresponding to the first period in the cardiac phase, the second doppler shift experienced by the second series of ultrasound waves.

At step 507 the processor 140 calculates a first velocity growth factor based on the first and second doppler shifts at the first and second corresponding periods in the cardiac phases.

The processor 140 then either:
at step 508a, determines that the user that the user is exercising below the anaerobic threshold if the first velocity growth factor is below the anaerobic velocity growth factor; or
at step 508b, determines that the user that the user is exercising at or above the anaerobic threshold if the first velocity growth factor is greater than or equal to the anaerobic velocity growth factor.

If step 508a is performed, the processor 140 then performs step 509a wherein the ultrasound transducer 110 sends a third series of ultrasound waves into the wrist 200 of the user at a doppler angle of between 0 and 80 degrees to a blood flow which are reflected, with a third doppler shift, back to the ultrasound transducer 110.

At step 510a the processor 140 determines, at a third period corresponding to the first period in the cardiac phase, the third doppler shift experienced by the third series of ultrasound waves. At step 511a the processor 140 calculates a second velocity growth factor based on the third doppler shift and the first doppler shift, and either:
at step 512a' determines that the user is exercising below the anaerobic threshold if the second velocity growth factor is below the anaerobic velocity growth factor; or
at step 512a" determines that the user that the user is exercising at or above the anaerobic threshold if the second velocity growth factor is greater than or equal to the anaerobic velocity growth factor.

If the processor 140 performs step 512a', the processor 140 then repeats the steps within box R and continues to send a new series of ultrasound waves into the wrist 200 of the user at a doppler angle of between 0 and 80 degrees to a blood flow which are reflected, with a doppler shift, back to the ultrasound transducer 110. The processor 140 determines the new associated doppler shift, at a period corresponding to the first period in the cardiac phase, experienced by each new series of ultrasound waves, and calculates the velocity growth factor based on the most recently calculated doppler shift and the first doppler shift, and either: determines that the user is exercising below the anaerobic threshold if the velocity growth factor is below the anaerobic velocity growth factor; or determines that the user that the user is exercising at or above the anaerobic threshold if the velocity growth factor is greater than or equal to the anaerobic velocity growth factor. The steps in box R are repeated such that the user can be alerted when the anaerobic threshold has been reached.

It will be understood that step 503, wherein the processor 140 calculates the first doppler shift experienced by the first series of ultrasound waves, may be performed later in the method, for example after step 504, wherein the user begins exercising with the device 100 attached to the wrist 200 and the device 100 registers that exercise has begun. In some examples, step 503 may be combined with step 506, such that the processor 140 determines the doppler shift of both the first series of waves and the second series of ultrasound waves at the same method step.

In this connection, it is not critical that the processor 140 determines the first doppler shift directly after the first series of ultrasound waves have been reflected back to the transducer.

The steps performed, both physical and by the processor 140, to determine when the user is exercising below or at or above the anaerobic threshold using the device 100 according to the second embodiment of the present disclosure are now explained, with reference to Fig. 14 already discussed.

When the device 100 is configured according to the second embodiment of the present disclosure, the steps performed are similar to those described in Fig. 14, except for the following:
at step 507 the processor 140 calculates a first velocity growth factor based on the first and second doppler shifts, the first and second ultrasound wave transmit frequencies and the doppler angle of the ultrasound waves;
if step 511a is performed, the processor 140 calculates a second velocity growth factor based on the third doppler shift and the first doppler shift, the associated ultrasound wave frequencies and the doppler angle of the ultrasound waves; and
if the processor 140 performs step 512a', the processor 140 then repeats the steps within box R and continues to send a new series of ultrasound waves into the wrist 200 of the user at a doppler angle of between 0 and 80 degrees to a blood flow which are reflected, with a doppler shift, back to the ultrasound transducer 110. The processor 140 determines the new associated doppler shift experienced by each the new series of ultrasound waves, and calculates the velocity growth factor based on the most recently calculated doppler shift and the first doppler shift, the associated ultrasound wave transmit frequencies and the doppler angle of the ultrasound waves, and either: determines that the user is exercising below the anaerobic threshold if the velocity growth factor is below the anaerobic velocity growth factor; or determines that the user that the user is exercising at or above the anaerobic threshold if the velocity growth factor is greater than or equal to the anaerobic velocity growth factor. The steps in box R are repeated such that the user can be alerted when the anaerobic threshold has been reached.

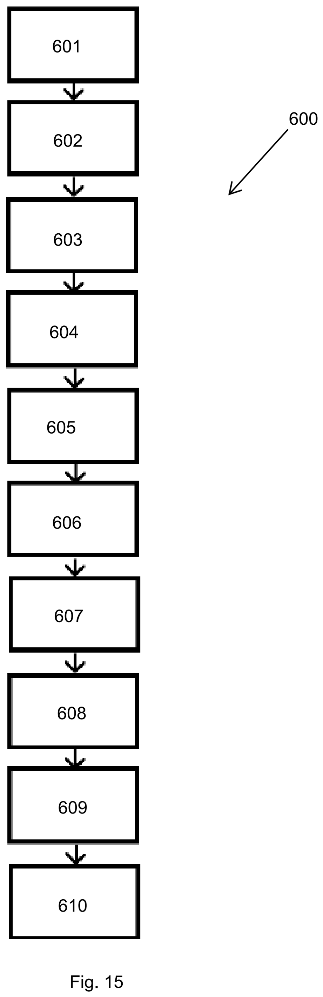

Referring now to Fig. 15, a flow diagram 600 is provided to explain the steps performed, both physical and by the processor 140, to determine the anaerobic threshold of the user according to the third embodiment of the present disclosure.

The flow diagram 600 starts at step 601, wherein the device 100 is attached to the wrist 200 of the user.

At step 602, while the user is exercising at a first intensity level, the ultrasound transducer 110 sends a first series of ultrasound waves into the wrist 200 of the user at a doppler angle of between 0 and 80 degrees which are reflected, with a first doppler shift, back to the ultrasound transducer 110.

At step 603 the processor 140 determines the first doppler shift experienced by the first series of ultrasound waves at a period in the cardiac phase.

At step 604, while the user is exercising at a second intensity level, the ultrasound transducer 110 sends a second series of ultrasound waves into the wrist 200 of the user at a doppler angle of between 0 and 80 degrees which are reflected, with a second doppler shift, back to the ultrasound transducer 110.

At step 605 the processor 140 determines the second doppler shift experienced by the second series of ultrasound waves at a corresponding period in the cardiac phase.

At step 606 the processor 140 calculates a first velocity growth factor based on the first and second doppler shifts at corresponding periods in the cardiac phases.

At step 607, while the user is exercising at a third intensity level, the ultrasound transducer 110 sends a third series of ultrasound waves into the wrist 200 of the user at a doppler angle of between 0 and 80 degrees which are reflected, with a third doppler shift, back to the ultrasound transducer 110.

At step 608 the processor 140 determines the third doppler shift experienced by the third series of ultrasound waves at a corresponding period in the cardiac phase.

At step 609 the processor 140 calculates a second velocity growth factor based on the second and third doppler shifts at corresponding periods in the cardiac phases.

At step 610 the processor 140 compares the second velocity growth factor with the first velocity growth factor and determines that the user is exercising at their anaerobic threshold when the difference between the first and second velocity growth factors is greater than a predetermined value. The anaerobic threshold for that user is then this point.

The first, second and third exercise intensity levels are required to be different from each other. The first, second and third exercise intensity levels need not be increasing step-wise.

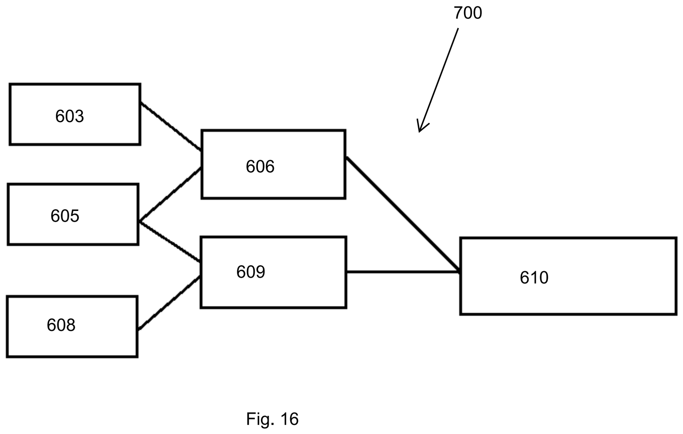

Fig. 16 shows an illustration of steps 603, 605, 606, 608, 609 and 610. As explained above, the device is attached to the wrist 200 of the user and a first series of ultrasound waves are sent at a doppler angle of between 0 and 80 degrees and reflected. At step 603 the processor 140 determines the first doppler shift at a period in the cardiac phase. A second series of ultrasound waves are sent at a doppler angle of between 0 and 80 degrees and reflected. At step 605 the processor 140 determines the second doppler shift at a corresponding period in the cardiac phase. At step 606 the processor 140 calculates a first velocity growth factor based on the first and second doppler shifts at corresponding periods in the cardiac phase, as shown in the illustration in Fig. 16.

A third series of ultrasound waves are sent at a doppler angle of between 0 and 80 degrees and reflected. At step 608 the processor 140 determines the third doppler shift at a corresponding period in the cardiac phase.

At step 609 the processor 140 calculates a second velocity growth factor based on the second and third doppler shifts at corresponding periods in the cardiac phase, as illustrated in Fig. 16.

At step 610 the processor 140 compares the second velocity growth factor with the first velocity growth factor and determines that the user is exercising at their anaerobic threshold when the difference between the first and second velocity growth factors is greater than a predetermined value. The anaerobic threshold is then at this point.

It will be understood that if there is not a significant difference between the first and second velocity growth factors, the ultrasound transducer 110 will send further series of ultrasound waves at a doppler angle of between 0 and 80 degrees, the processor 140 will determine the doppler shift of the reflected waves at a corresponding period of the cardiac phase, and calculate a velocity growth factor as discussed above. A comparison between the velocity growth factors will again be made and the processor 140 will again determine that the user is exercising at the anaerobic threshold when there is a significant difference in the two velocity growth factors. The anaerobic threshold for that user is then determined to be at this point.

As described throughout the present description, there are many different arrangements possible involving the separation or combination of components such as the ultrasound transducer 110, display (where provided), processor 140 etc. It will be understood by a person skilled in the art that the specific arrangement, locations and connections between these components is not important for the performance of the invention.

The minimum components required to provide the third embodiment of the present disclosure are a device 100 for determining the anaerobic threshold, the device comprising an ultrasound transducer 110 and a processor 140. The ultrasound transducer 110 is signal connected to the processor 140 and the ultrasound transducer 110 is arranged in use to: be attached to the body of the user during exercise; send successive ultrasound waves to a blood flow in the body at a doppler angle of between 0 and 80 degrees; receive successive ultrasound wave reflections back from the blood flow with a doppler shift; and send the ultrasound wave data to the processor 140; wherein the processor 140 is configured to: receive ultrasound wave data from the ultrasound transducer 110 and determine the doppler shift of the ultrasound waves sent and reflected off of the blood flow; from the doppler shift of successive waves, determine the velocity growth factor of the blood flow; and determine the anaerobic threshold as the point when the velocity growth factor changes beyond a tolerance value.

Throughout the present disclosure it is stated that the doppler angle, i.e. the angle between the ultrasound beam and the blood flow must be between 0 and 80 degrees. This is to minimise errors. Since the cosine of the angle is used in calculations, errors will increase significantly at larger angles. It will be understood that in other literature the doppler angle may be referred to as the angle of insonation.

Although not described in the present description in the interest of brevity, it will be understood that the device may be provided with the required memory such that it can temporarily store data for processing. For example, in the first, second and third embodiments, the device may be required to temporarily store the data relating to the rest state for use in future calculations.

### CLAUSES

A selection of numbered clauses is now presented.

Clause 1. A device (100) for determining in use when a user is exercising below, at or above the anaerobic threshold, the device (100) comprising an ultrasound transducer (110) and a processor (140) wherein:
the ultrasound transducer (110) is signal connected to the processor (140) and the ultrasound transducer (110) is arranged in use to:
be attached to the body of the user before and during exercise;
send a series of ultrasound waves at a doppler angle (θ) of between 0 and 80 degrees to a blood flow (210) in the body before exercise begins;
receive a first series of ultrasound wave reflections back from the blood flow (210) with a doppler shift; and
send a series of ultrasound waves at the same doppler angle (θ) of between 0 and 80 degrees to a blood flow (210) in the body during exercise;
receive a second series of ultrasound wave reflections back from the blood flow (210) with a doppler shift; and
send the ultrasound wave data to the processor (140);
wherein the processor (140) is configured to:
receive ultrasound wave data from the ultrasound transducer (110) and:
determine, at a first period in the cardiac phase, the doppler shift of the series of ultrasound waves sent to the blood flow (210) before exercise;
determine, at a second period in the cardiac phase corresponding to the first period in the cardiac phase, the doppler shift of the series of ultrasound waves sent to the blood flow (210) during exercise; and
calculate a velocity growth factor based on the doppler shifts at the first and second corresponding periods in the cardiac phases; and
compare the velocity growth factor with a predetermined anaerobic velocity growth factor or a predetermined anaerobic velocity growth factor range; and
determine that the user is:
   exercising below the anaerobic threshold if the velocity growth
   factor is less than the anaerobic velocity growth factor or less than the lower limit of the anaerobic velocity growth factor range; or
   exercising at the anaerobic threshold if the velocity growth factor is equal to the anaerobic velocity growth factor or within the anaerobic velocity growth factor range; or
   exercising above the anaerobic threshold if the velocity growth factor is greater than the anaerobic velocity growth factor or greater than the upper limit of the anaerobic velocity growth factor range.

Clause 2. The device (100) according to clause 1, wherein:
the ultrasound transducer (110) is further configured to:
send the series of ultrasound waves to a blood flow (210) in the body before exercise begins at a first frequency (*f*₁); and
send the series of ultrasound waves to a blood flow (210) in the body during exercise at a second frequency (*f*₂);
and the processor (140) is further configured to calculate the velocity growth factor further based on the first (*f*₁) and second (*f*₂) frequencies and the doppler angle (θ).

Clause 3. The device (100) according to clause 1 or 2, further comprising a button (130) configured to send a signal to the processor (140) that exercise has started when the button (130) has been pressed by the user.

Clause 4. The device (100) according to any of clauses 1 to 3 further comprising an attachment means configured to hold the ultrasound transducer (110) attached to the body of the user in use.

Clause 5. A method of determining when a user is exercising below, at or above the anaerobic threshold, the method comprising the steps of:
a) providing a device (100) according to clause 1;
b) attaching the ultrasound transducer (110) to the body of the user before the user begins exercising;
c) sending a first series of ultrasound waves at a doppler angle (θ) of between 0 and 80 degrees to a blood flow (210) in the body while the user is not exercising;
d) receiving a first series of ultrasound wave reflections back from the blood flow (210) with a doppler shift; and
e) sending a second series of ultrasound waves at the same doppler angle (θ) of between 0 and 80 degrees to a blood flow (210) in the body during exercise;
f) receiving a second series of ultrasound wave reflections back from the blood flow (210) with a doppler shift; and
g) sending the first ultrasound wave data to the processor (140);
h) sending the second ultrasound wave data to the processor (140);
i) determining, at a first period in the cardiac phase, the doppler shift of the first series of waves;
j) determining, at a second period corresponding to the first period in the cardiac phase, the doppler shift of the second series of waves;
k) calculating a velocity growth factor based on the doppler shifts at the first and second corresponding periods in the cardiac phases;
l) comparing the velocity growth factor with a predetermined anaerobic velocity growth factor or a predetermined anaerobic velocity growth factor range; and
m) determining that the user is:
   i. exercising below the anaerobic threshold if the velocity growth factor is less than the anaerobic velocity growth factor or less than the lower limit of the anaerobic velocity growth factor range; or
   ii. exercising at the anaerobic threshold if the velocity growth factor is equal to the anaerobic velocity growth factor or within the anaerobic velocity growth factor range; or
   iii. exercising above the anaerobic threshold if the velocity growth factor is greater than the anaerobic velocity growth factor or greater than the upper limit of the anaerobic velocity growth factor range.

Clause 6. The method according to clause 5, wherein:
the step of sending a first series of ultrasound waves further comprises sending the first series of ultrasound waves at a first frequency (*f*₁);
the step of sending a second series of ultrasound waves further comprises sending the second series of ultrasound waves at a second frequency (*f*₂); and
the step of calculating the velocity growth factor is further based on the first (*f*₁) and second (*f*₂) frequencies and the doppler angle (θ).

Clause 7. The method according to clause 5 or 6 wherein the method further comprises the step of:
d1) the processor (140) detecting that exercise has started;
wherein step d1 is performed between steps d) and e).

Clause 8. The method according to clause 7, wherein the step of the processor (140) detecting that exercise has started comprises the user providing an input to the processor (140) to indicate that exercise has started

Clause 9. The method according to any of clauses 5 to 8 wherein either:
step g. is performed before step e; or
step g. and step i. are both performed before step e.

Clause 10. The method according to any of clauses 5 to 9, further comprising, after step m, repeating steps e, f, h, j, k, I and m again.

Clause 11. A device (100) for determining the anaerobic threshold of a user, the device (100) comprising an ultrasound transducer (110) and a processor (140) wherein:
the ultrasound transducer (110) is signal connected to the processor (140) and the ultrasound transducer (110) is configured in use to:
be attached to the body of the user;
send a first, a second and a third series of ultrasound waves at the same doppler angle (θ) of between 0 and 80 degrees to a blood flow (210), wherein the first, second and third series of ultrasound waves can be sent to the body during exercise wherein each series of ultrasound waves can be sent when the body is at different exercise intensities;
receive first, second and third series of ultrasound wave reflections back from the blood flow (210) with a doppler shift; and
send the ultrasound wave data to the processor (140);
wherein the processor (140) is configured to:
receive ultrasound wave data from the ultrasound transducer (110) and:
determine, at corresponding periods in the cardiac phase, the doppler shift of each the three series of ultrasound waves sent to the blood flow (210);
calculate a first velocity growth factor based on the doppler shifts of the first and second series of ultrasound waves at corresponding cardiac phases of the series;
calculate a second velocity growth factor based on the doppler shifts of the second and third series of ultrasound waves at corresponding cardiac phases of the series;
compare the first and second velocity growth factors; and
determine the anaerobic threshold of the user as the point where the difference between the first and second velocity growth factors is greater than a predetermined value.

Clause 12. A method of determining the anaerobic threshold of a user, comprising the steps of:
a) providing a device (100) according to clause 11;
b) attaching the device (100) to the body of a user;
c) sending a first, a second and a third series of ultrasound waves at the same doppler angle (θ) of between 0 and 80 degrees to a blood flow (210) in the body during exercise, wherein each series of ultrasound waves is sent at different exercise intensities;
d) receiving a first, a second and a third series of ultrasound wave reflections back from the blood flow (210) with a doppler shift; and
e) sending the ultrasound wave data to the processor (140);
f) determining, at corresponding periods in the cardiac phases, the doppler shift of each of the three series of waves;
g) calculating a first velocity growth factor based on the doppler shifts of the first and second series of ultrasound waves at corresponding periods in the cardiac phases of the series;
h) calculating a second velocity growth factor based on the doppler shift of the second and third series of ultrasound waves at corresponding periods in the cardiac phases of the series;
i) comparing the first and second velocity growth factors; and
j) determining the anaerobic threshold of the user as the point where the difference between the velocity growth factors is greater than a predetermined value.

Clause 13. The method according to clause 12, wherein the method further comprises, after step i) and before step j):
- determining that the difference between the first and second velocity growth factors is not greater than a predetermined value,
   - sending further series of ultrasound waves at the same doppler angle (θ) of between 0 and 80 degrees to a blood flow (210) in the body during exercise and different exercise intensities from the previous series of ultrasound waves;
   - receiving further series of ultrasound wave reflections back from the blood flow (210) with a doppler shift; and
   - sending the ultrasound wave data to the processor (140);
- determining, at corresponding periods in the cardiac phases, the doppler shift of each of the further series of waves;
- calculating further velocity growth factors based on the doppler shifts at corresponding periods in the cardiac phases of the further series of ultrasound waves; and
   - comparing the velocity growth factors.

## Claims

1. A device (100) for determining the anaerobic threshold of a user, the device (100) comprising an ultrasound transducer (110) and a processor (140) wherein:
the ultrasound transducer (110) is signal connected to the processor (140) and the ultrasound transducer (110) is configured in use to:
be attached to the body of the user;
send a first, a second and a third series of ultrasound waves at the same doppler angle (θ) of between 0 and 80 degrees to a blood flow (210), wherein the first, second and third series of ultrasound waves can be sent to the body during exercise wherein each series of ultrasound waves can be sent when the body is at different exercise intensities;
receive first, second and third series of ultrasound wave reflections back from the blood flow (210) with a doppler shift; and
send the ultrasound wave data to the processor (140);
wherein the processor (140) is configured to:
receive ultrasound wave data from the ultrasound transducer (110) and:
determine, at corresponding periods in the cardiac phase, the doppler shift of each the three series of ultrasound waves sent to the blood flow (210);
calculate a first velocity growth factor based on the doppler shifts of the first and second series of ultrasound waves at corresponding cardiac phases of the series, wherein the first velocity growth factor means the ratio of the doppler shift of a second series of ultrasound waves relative to the doppler shift of a third series of ultrasound waves;
calculate a second velocity growth factor based on the doppler shifts of the second and third series of ultrasound waves at corresponding cardiac phases of the series, wherein the second velocity growth factor means the ratio of the doppler shift of a third series of ultrasound waves relative to the doppler shift of a second series of ultrasound waves;
compare the first and second velocity growth factors; and
determine the anaerobic threshold of the user as the point where the difference between the first and second velocity growth factors is greater than a predetermined value.

2. A method of determining the anaerobic threshold of a user, comprising the steps of:
a) providing a device (100) according to claim 1;
b) attaching the device (100) to the body of a user;
c) sending a first, a second and a third series of ultrasound waves at the same doppler angle (θ) of between 0 and 80 degrees to a blood flow (210) in the body during exercise, wherein each series of ultrasound waves is sent at different exercise intensities;
d) receiving a first, a second and a third series of ultrasound wave reflections back from the blood flow (210) with a doppler shift; and
e) sending the ultrasound wave data to the processor (140);
f) determining, at corresponding periods in the cardiac phases, the doppler shift of each of the three series of waves;
g) calculating a first velocity growth factor based on the doppler shifts of the first and second series of ultrasound waves at corresponding periods in the cardiac phases of the series, wherein the first velocity growth factor means the ratio of the doppler shift of a second series of ultrasound waves relative to the doppler shift of a third series of ultrasound waves;
h) calculating a second velocity growth factor based on the doppler shift of the second and third series of ultrasound waves at corresponding periods in the cardiac phases of the series, wherein the second velocity growth factor means the ratio of the doppler shift of a third series of ultrasound waves relative to the doppler shift of a second series of ultrasound waves;
i) comparing the first and second velocity growth factors; and
j) determining the anaerobic threshold of the user as the point where the difference between the velocity growth factors is greater than a predetermined value.

3. The method according to claim 2, wherein the method further comprises, after step i) and before step j):
- determining that the difference between the first and second velocity growth factors is not greater than a predetermined value,
- sending further series of ultrasound waves at the same doppler angle (θ) of between 0 and 80 degrees to a blood flow (210) in the body during exercise and different exercise intensities from the previous series of ultrasound waves;
- receiving further series of ultrasound wave reflections back from the blood flow (210) with a doppler shift; and
- sending the ultrasound wave data to the processor (140);
- determining, at corresponding periods in the cardiac phases, the doppler shift of each of the further series of waves;
- calculating further velocity growth factors based on the doppler shifts at corresponding periods in the cardiac phases of the further series of ultrasound waves; and
- comparing the velocity growth factors.
